# EUROPEAN PATENT SPECIFICATION

(11) **EP 0 481 257 B1**
(45) Date of publication and mention of the grant of the patent: **06.12.1995**
(21) Application number: 91116381.4
(22) Date of filing: 26.09.1991
(51) Int. Cl.: A61M 1/16

(54) **Method and system for preparation of a medical solution, for example a dialysis solution**
Verfahren und System zur Herstellung von medizinischen Lösungen, insbesondere zur Dialyselösung
Procédé et système pour la préparation d'une solution médicale, par exemple une solution de dialyse

(30) Priority: 15.10.1990 SE 9003278
(43) Date of publication of application: 22.04.1992
(73) Proprietor: GAMBRO AB, S-220 10 Lund (SE)
(72) Inventor: Jönsson, Lennart, S-240 20 Furulund (SE); Jönsson, Sven, S-245 00 Staffanstorp (SE)
(74) Representative: Asketorp, Göran

(56) References cited:
- EP-A- 0 209 607
- PATENT ABSTRACTS OF JAPAN, vol. 6, no. 49 (C-096), 31st March 1982; & JP-A-56 164 113 (DAIGO MINORU) 17-12-1981

## Description

### TECHNICAL FIELD

The present invention relates to a method for preparation of a medical solution, for example a dialysis solution, from substantially water and a plurality of concentrates of substances to be included in said medical solution, whereby respective concentrates are supplied successively to a number of dosage points along a main conduit extending from a water source to a place of consumption, such as a dialyzer.

The invention also relates to an apparatus for preparation of a medical solution, for example a dialysis solution, from substantially water and a plurality of concentrates of substances to be included in said medical solution, whereby respective concentrates are arranged to be supplied successively to a number of dosage points along a main conduit extending from a water source to a place of consumption, such as a dialyzer.

The invention is preferably intended to be applied to dialysis systems in which a first concentrate comprises essentially only bicarbonate and a second concentrate comprises among others a calcium salt, preferably calcium chloride.

### BACKGROUND ART

A method for preparation of a dialysis solution is described in, for example, EP-B1-0 022 922, in which two concentrates are used, one of which comprising sodium bicarbonate as a buffer and the other comprising metal salts and an acid.

A more elaborate system for preparation of a medical solution, preferably intended for dialysis, is described in US-A-4 784 495, in which concentrates in liquid form are partly replaced with one or more concentrates in powder form. However, at least one concentrate in liquid form must still be used, which is a drawback.

In patent abstracts of Japan JP-A-56-164113, there is disclosed a method for preparation of a dialysis solution from water and two concentrates, one of which comprising bicarbonate. Carbon dioxide gas is introduced into a water supply vessel for adjusting the pH-value of the water to a low value so that precipitation of carbonate salts is prevented in the dialysis solution. By adding carbon dioxide gas to the water supply vessel, which also is heated, the content of the water supply vessel will be corrosive and destroy the vessel and adjacent lines unless made by corrosion resistant materials, which are expensive. Moreover, simultaneous heating and supply of carbon dioxide gas will not promote solution of the carbon dioxide gas in water, since the solubility is inversely proportional to the temperature.

### DESCRIPTION OF THE INVENTION

A main object of the present invention is to make possible complete elimination of concentrates in liquid form in the preparation of dialysis solution. The only liquid required is water. By using concentrate in powder form, dissolved in situ, bacteria growth is normally considerably less than in a corresponding concentrate in liquid form.

However, the invention may also be applied in combination with methods and apparatuses in which some concentrates, for various reasons are supplied in liquid form.

A more detailed object of the present invention is to prevent precipitation of calcium carbonate when bicarbonate and calcium cloride is mixed during preparation of the medical solution and subsequent use. In such a case, the addition of the acid in gas form should take place before these substances are blended.

A further object of the present invention is to provide a concentrate in the form of a pure acid. As such, no change of the concentration needs to take place for the metal salts added via respective concentrates.

A still further object of the invention is to be able to control the addition of the acid in gas form to be sure that sufficient gas has been introduced into the solution.

For fulfilling the above mentioned object, there is disclosed a method for preparation of a medical solution, for example a dialysis solution, from substantially water and a plurality of concentrates of substances to be included in said medical solution. The method comprises the steps of supplying substantially water from a source to a main conduit; dosing a first concentrate of said plurality of concentrates to a first mixing point of said main conduit for mixing with the content of said main conduit for obtaining a presolution with a predetermined concentration of substances of said first concentrate; dosing a second concentrate of said plurality of concentrates to a second mixing point of said main conduit for mixing with the content of said main conduit for obtaining a presolution with a predetermined concentration of substances of said second concentrate in said water; optionally dosing further concentrates to further mixing points of said main conduit for mixing with the content of said main conduit; for forming said medical solution to be supplied to a medical device, for example a dialyzer.

According to the invention the method is characterized in supplying an acid in gas form to an intermediate mixing position between said first mixing position and said second mixing position, whereby one of said first and second concentrates comprises bicarbonate, preferably sodium bicarbonate, and the other comprises a calcium salt, preferably calcium chloride.

Moreover, there is disclosed an apparatus for preparation of a medical solution, for example a dialysis solution, from substantially water and a plurality of concentrates of substances to be included in said medical solution. The apparatus comprises an inlet for supplying substantially water from a source to a main conduit; a first mixing point of said main conduit for receiving a first concentrate of said plurality of concentrates to be mixed with the content of said main conduit for obtaining a presolution with a predetermined concentration of substances of said first concentrate; a second mixing point of said main conduit for receiving a second concentrate of said plurality of concentrates to be mixed with the content of said main conduit for obtaining a presolution with a predetermined concentration of substances of said second concentrate in said water; optionally further mixing points of said main conduit for receiving further concentrates to be mixed with the content of said main conduit; for forming said medical solution to be supplied to a medical device, for example a dialyzer.

The apparatus is characterized by an intermediate mixing point between said first mixing point and said second mixing point for receiving an acid in gas form, whereby one of said first and second concentrates comprises bicarbonate, preferably sodium bicarbonate, and the other comprises a calcium salt, preferably calcium chloride.

Preferably, the gas is supplied in excess and surplus gas is removed in a subsequent degasing step, whereupon the removed gas optionally is recirculated. This can be desirable, partially from an economic point of view and partially for facilitating the control of the finally supplied quantity of gas.

For controlling the supply of gas, a property of the solution is monitored after the addition of said gas. Such property can be the pH-value of the solution or the conductivity thereof.

Considerable advantages, such a from a transportation point of view, are gained if the gas is prepared in situ. If this concerns a dialysis system which uses sodium bicarbonate as the buffer, then it is suitable that carbon dioxide gas, CO_{2,} is chosen as such a gas.

According to a preferred embodiment of the invention, the acid is prepared in situ, whereby the acid preferably is carbon dioxide gas, CO₂. Such preparation can involve mixing carbonate, preferably sodium bicarbonate, with water and acid, preferably citric acid. Another preparation method is by heating a carbonate, preferably sodium bicarbonate, to a predetermined temperature, for example 50°C. Preferably, water formed during the preparation, is separated before supply to the main conduit. From a hygienic view point, it can be appropriate that water formed during the preparation is separated off before the supply to the main line.

The invention also relates to a method and apparatus for continuous preparation of a medical solution, for example a dialysis solution from water and a plurality of concentrates, by adding a first concentrate comprises metal salts to be included in the medical solution, among others calcium chloride, and a second concentrate comprises sodium bicarbonate obtained from feeding water through a dry powder of sodium bicarbonate to water in a continuous process.

In such a case, the dosage inlet point for the acid in gas form should be positioned between the dosage points for these substances. By the addition between the two introduction and mixing positions, it is assured that no gas can escape to the surroundings until a subsequent degasing step.

Alternatively, other gases can be used in the present invention, such as hydrochloric acid in gas form.

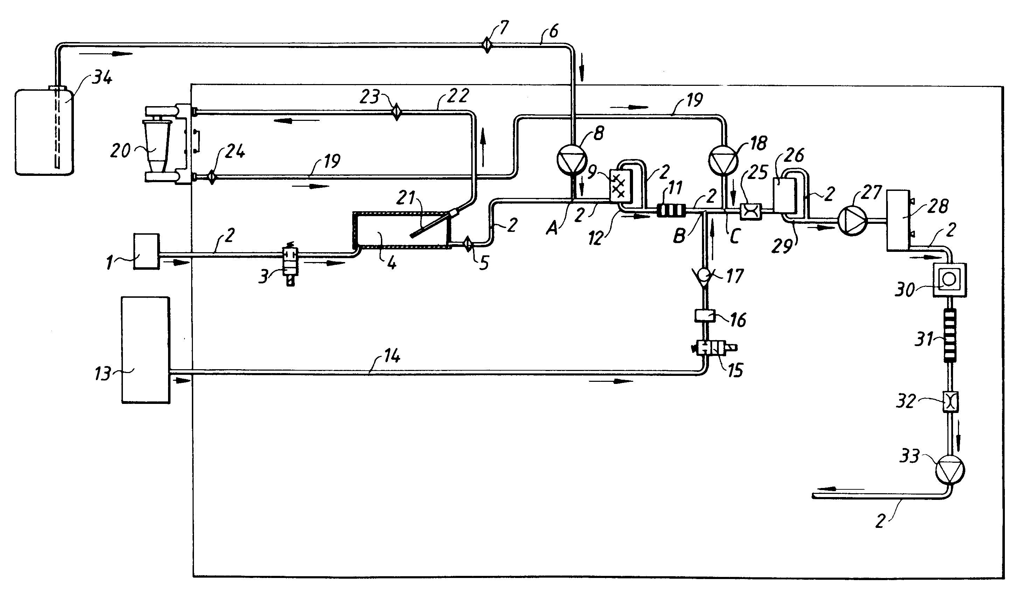

### BRIEF DESCRIPTION OF THE DRAWING

In the attached drawing a system or a plant for preparation of a medical solution, preferably a dialysis solution, is shown in simple block form. For the sake of clarity, less important details for the invention have been omitted.

### PREFERRED EMBODIMENT OF A SYSTEM ACCORDING TO THE INVENTION

A preferred embodiment of a system according to the invention is hereby shown in the drawing. In the drawing, water is supplied from a source 1, for example a reverseosmosis unit. Alternatively, the source 1 can constitute a hospital's central system in which water has already been supplied with one or more concentrates in fixed concentrations. In the following, the expression water source is meant not solely a source for pure water, but also sources for water to which one or more substances have been added. The water is fed via a main conduit 2 with a valve 3 to a heating vessel 4, where it is heated to a temperature, for example circa 37°C. The main conduit 2 then continues via a filter 5 to a mixing point A. A liquid-based concentrate is supplied from a reservoir 34 via a conduit 6 with a filter 7. Alternatively, this concentrate can be supplied in powder form and dissolved in situ by water from the water source 1 being fed therethrough, as is described for example in the above-mentioned American patent 4 784 495 or in the Swedish patent application 90.00586-9. The concentrate from the source 34 or concentrate prepared in the abovementioned way is supplied to the main conduit 2 with the aid of a pump 8.

In order to achieve good mixing, the water and concentrate are fed to a mixing vessel 9 and from there to a conductivity meter 11. So that the mixing vessel 9 can be emptied after treatment has been carried out, it is provided with a separate drainage conduit 12 at its base. The conductivity meter 11 is appropriately arranged to control the pump 8 in order to achieve the correct mixing ratio between water and concentrate.

According to the invention, a concentrate is supplied to the main conduit 2 in the form of a gaseous acid. This acid is drawn from a reservoir 13 therefor. This reservoir 13 can either consist of a gas bottle with suitable gas, for example carbon dioxide or HCl in gas form. Alternatively, it can consist of a device for preparing the gas in situ. By way of example, CO₂ can be prepared in situ by means of a carbonate, preferably sodium carbonate, being mixed with acid and water. An example of a suitable acid for this purpose is citric acid. Alternatively, CO₂ can be prepared by warming a carbonate, preferably sodium carbonate, to a suitable temperature, such as over 50°C.

The gas from the reservoir 13 is then led through a conduit 14, a valve 15, a flow regulation valve 16 and a non-return valve 17 to a mixing point B in the main conduit 2.

A third concentrate from a reservoir 20 in the form of a powder cartridge is then supplied to a point C in the main conduit with the aid of a pump 18 via conduit 19. The powder therein is dissolved continuously by water being drawn from the heating vessel via a tube 21 and a conduit 22 with a filter 23. The conduit 19 also includes a filter which is designated by 24. The dissolving of the powder in the cartridge 20 can occur in a way as described in the above-mentioned American patent 4 784 495.

The prepared liquid is delivered from the mixing point C via a throttle 25 and bubble chamber 26 with the aid of a pump 27 to a bubble trap 28. Through this arrangement, bubbles are formed from mainly air dissolved in the liquid and any surplus gas supplied from the reservoir 13. These bubbles are enlarged in the bubble chamber 26 and removed in the bubble trap 28 in a not shown way. These, together with a smaller quantity of liquid, can possibly be led directly to a drain or may also be recirculated so that surplus gas can also be dissolved. Like the mixing vessel 9, the bubble chamber 26 is also provided with a drainage conduit 29 at its base. Thus, this can be completely emptied when the system for example is to be cleaned.

The main conduit 2 extends from the bubble trap 28 via a pH-meter 30, a conductivity meter 31, a throttle 32 and a pump 33 to a not shown dialyzer. The pH-meter 30 is hereby suitably arranged to control the flow regulator 16 in the conduit 14. The conductivity meter 31 is further suitably arranged to control the pump 18 in the conduit 19. These pumps can alternatively consist of some type of adjustable dosage pump to which a suitable value can be inputted in relation to the flow of liquid through the main conduit 2.

The components 32 and 33 can be parts of a constant flow device of the type which is described in more detail in the American patent 4 762 618. In such a case, a pressure meter is provided therebetween which controls the pump 33 so that a constant pressure drop is maintained across the throttle 32 and thereby a constant flow to the dialyzer.

### EXAMPLE FOR PREPARATION OF CO₂

### 1. Sodium bicarbonate mixed with acid and water

Acid in dry form, such as citric acid, is mixed in suitable quantities with bicarbonate. For dialysis treatment the suitable quantities are circa 37 g citric acid and 45 g bicarbonate. When water is added, the formation of CO₂ commences. In order to moderate the supply of gas, either the water supply to the powder mixture can be regulated and thereby control the gas production, or the gas can be allowed to be collected in a reservoir under pressure and the supply of gas to the main conduit can be regulated with a flow regulating device.

### 2. Sodium bicarbonate heated to conversion temperature

Dry sodium bicarbonate is heated to a temperature above 50°C in a gas-tight vessel which is connected to the point of consumption. The gas production is directly proportional to this supply of energy. In this way, the gas production can be controlled as needed. In order to control the quantity of supplied energy, either the gas flow or the pH-value in the ready dialysis liquid can be measured.

### DETAIL INFORMATION

Surplus gas can either be separated off in connection with the normal degassifying or can be brought to recirculate in the system.

Parameters which effect the quantity of supplied acid are, amongst others, the pressure at the dosage intake point, the temperature of the liquid, exposure time for the gas (contact distance/flow velocity) and contact surface (total bubble surface).

Naturally the invention is not restricted to solely the above-described embodiment, but can be varied within the scope of the appended claims. By way of example, other gases such as hydrochloric acid in gas form can be used. The hydrochloric acid need not be in gas form from the outset. Instead this can be generated in situ by mixing for example sodium chloride with a suitable acid, such as sulphuric acid, during heating. However, it is preferable to use CO₂. Furthermore, it should be noted that the conductivity meter 31 can be arranged solely for control of the preparation. In such a case, the flow control valve 16 can totally control the gas flow in the conduit 14. Besides regulating means, in such a case this can also include means for measuring the flow. Furthermore, the parts included in the described system can be varied within wide limits concerning both form and function.

## Claims

1. Method for preparation of a medical solution, for example a dialysis solution, from substantially water and a plurality of concentrates of substances to be included in said medical solution, said method comprising the steps of:
supplying substantially water from a source (1) to a main conduit (2);
dosing a first concentrate (34) of said plurality of concentrates to a first mixing point (A) of said main conduit (2) for mixing with the content of said main conduit (2) for obtaining a presolution with a predetermined concentration of substances of said first concentrate;
dosing a second concentrate (20) of said plurality of concentrates to a second mixing point (C) of said main conduit (2) for mixing with the content of said main conduit (2) for obtaining a presolution with a predetermined concentration of substances of said second concentrate in said water;
optionally dosing further concentrates to further mixing points of said main conduit for mixing with the content of said main conduit (2);
for forming said medical solution to be supplied to a medical device, for example a dialyzer;
**characterized** in:
supplying an acid in gas form to an intermediate mixing position (B) between said first mixing position (A) and said second mixing position (C), whereby one of said first and second concentrates comprises bicarbonate, preferably sodium bicarbonate, and the other comprises a calcium salt, preferably calcium chloride.

2. Method according to claim 1, **characterized** in that surplus gas, after mixing, is removed in a degasing step, whereupon said removed gas optionally is recirculated.

3. Method according to anyone of the previous claims, **characterized** in monitoring a property of the solution after the addition of said gas, for example the pH-value of the solution, and controlling the supply of acid in gas form in dependence of the monitored property.

4. Method according to anyone of the previous claims, **characterized** in preparing said acid in gas form in situ, said acid in gas form preferably being carbon dioxide gas, CO₂.

5. Method according to claim 4, **characterized** in preparing said acid in gas form, i.e. carbon dioxide gas, CO₂, by mixing carbonate, preferably sodium bicarbonate, with water and acid, preferably citric acid.

6. Method according to claim 4, **characterized** in preparing said acid in gas form, i.e. carbon dioxide gas, CO₂, by heating a carbonate, preferably sodium bicarbonate, to a predetermined temperature, for example 50°C.

7. Method according to claim 6, **characterized** in separating water formed during the preparation, before supply to the main conduit.

8. Apparatus for preparation of a medical solution, for example a dialysis solution, from substantially water and a plurality of concentrates of substances to be included in said medical solution, said apparatus comprising:
an inlet for supplying substantially water from a source (1) to a main conduit (2);
a first mixing point (A) of said main conduit for receiving a first concentrate (34) of said plurality of concentrates to be mixed with the content of said main conduit (2) for obtaining a presolution with a predetermined concentration of substances of said first concentrate;
a second mixing point (C) of said main conduit for receiving a second concentrate (20) of said plurality of concentrates to be mixed with the content of said main conduit (2) for obtaining a presolution with a predetermined concentration of substances of said second concentrate in said water;
optionally further mixing points of said main conduit for receiving further concentrates to be mixed with the content of said main conduit (2);
for forming said medical solution to be supplied to a medical device, for example a dialyzer;
**characterized** by:
an intermediate mixing point (B) between said first mixing point (A) and said second mixing point (C) for receiving an acid in gas form, whereby one of said first and second concentrates comprises bicarbonate, preferably sodium bicarbonate, and the other comprises a calcium salt, preferably calcium chloride.

9. Apparatus according to claim 8, **characterized** by removing means (25,26,27,28) for removing surplus gas after mixing, and preferably also recirculation means for recirculating said removed gas.

10. Apparatus according to claim 8 or 9, **characterized** by a monitoring and control means (30) for monitoring a property, for example the pH-value, of the solution after the addition of said gas and controlling the supply of acid in gas form in dependence thereof.

11. Apparatus according to anyone of claims 8-10, **characterized** by an acid gas generator (13) for preparing said acid in gas form in situ, preferably carbon dioxide gas, CO₂.

12. Apparatus according to claim 11, **characterized** by means (13) for mixing carbonate, preferably sodium bicarbonate, with water and acid, preferably citric acid, for preparing carbon dioxide gas, CO₂.

13. Apparatus according to claim 11, **characterized** by means (13) for heating carbonate, preferably sodium bicarbonate, to a predetermined temperature, for example 50 C, for preparing carbon dioxide gas, CO₂.

14. Apparatus according to claim 13, **characterized** by separation means for separating water formed during the preparation before supply to the main conduit.

15. Apparatus according to anyone of claims 8-14, **characterized** in that said first concentrate (34) comprises metal salts to be included in the medical solution, among others calcium chloride, and that said second concentrate comprises sodium bicarbonate obtained from feeding water through a dry powder of sodium bicarbonate for continuous preparation of the medical solution.

## Patentansprüche

1. Verfahren zur Herstellung einer medizinischen Lösung, wie beispielsweise einer Dialyselösung, im wesentlichen aus Wasser und mehreren Konzentraten von Substanzen, die in die medizinische Lösung eingearbeitet werden sollen, mit den Stufen, in denen man
im wesentlichen Wasser von einer Quelle (1) einer Hauptleitung (2) zuführt,
ein erstes Konzentrat (34) der mehreren Konzentrate einem ersten Mischpunkt (A) der Hauptleitung (2) zudosiert, um es mit dem Inhalt der Hauptleitung (2) zu vermischen und so eine Vorlösung mit einer vorbestimmten Konzentration von Substanzen des ersten Konzentrates zu erhalten,
ein zweites Konzentrat (20) der mehreren Konzentrate einem zweiten Mischpunkt (C) der Hauptleitung (2) zudosiert, um es mit dem Inhalt der Hauptleitung (2) zu vermischen und so eine Vorlösung mit einer vorbestimmten Konzentration von Substanzen des zweiten Konzentrates in dem Wasser zu erhalten,
gegebenenfalls weitere Konzentrate weiteren Mischpunkten der Hauptleitung zudosiert, um sie mit dem Inhalt der Hauptleitung (2) zu vermischen, um die einer medizinischen Einrichtung, wie beispielsweise einem Dialysegerät, zuzuführende medizinische Lösung zu bilden,
**dadurch gekennzeichnet**, daß man eine Säure in Gasform einer Mischposition (B) zwischen der ersten Mischposition (A) und der zweiten Mischposition (C) zuführt, wobei eines der ersten und zweiten Konzentrate Bicarbonat, vorzugsweise Natriumbicarbonat, umfaßt und das andere ein Calciumsalz, vorzugsweise Calciumchlorid, umfaßt.

2. Verfahren nach Anspruch 1, **dadurch gekennzeichnet**, daß Überschußgas nach dem Vermischen in einer Entgasungsstufe entfernt wird, worauf das entfernte Gas gegebenenfalls rezirkuliert wird.

3. Verfahren nach einem der vorausgehenden Ansprüche, **dadurch gekennzeichnet**, daß man eine Eigenschaft der Lösung nach der Zugabe des Gases, wie beispielsweise den pH-Wert der Lösung, überwacht und die Zufuhr von Säure in Gasform in Abhängigkeit von der überwachten Eigenschaft steuert.

4. Verfahren nach einem der vorausgehenden Ansprüche, **dadurch gekennzeichnet**, daß man die Säure in Gasform in situ herstellt, wobei die Säure in Gasform vorzugsweise Kohlendioxidgas, CO₂, ist.

5. Verfahren nach Anspruch 4 **dadurch gekennzeichnet**, daß man die Säure in Gasform, d. h. Kohlendioxidgas, CO₂, durch Vermischen von Carbonat, vorzugsweise von Natriumbicarbonat, mit Wasser und Säure, vorzugsweise Zitronensäure, herstellt.

6. Verfahren nach Anspruch 4, **dadurch gekennzeichnet**, daß man die Säure in Gasform, d. h. Kohlendioxidgas, CO₂, durch Erwärmen eines Carbonats, vorzugsweise von Natriumbicarbonat, auf eine vorbestimmte Temperatur, wie beispielsweise 50 °C, herstellt.

7. Verfahren nach Anspruch 6, **dadurch gekennzeichnet**, daß man während der Herstellung gebildetes Wasser vor der Zufuhr zu der Hauptleitung abtrennt.

8. Vorrichtung zur Herstellung einer medizinischen Lösung, wie beispielsweise einer Dialyselösung, im wesentlichen aus Wasser und aus mehreren Konzentraten von Substanzen, die in die medizinische Lösung eingearbeitet werden sollen, mit
einem Einlaß zur Zufuhr von im wesentlichen Wasser aus einer Quelle (1) zu einer Hauptleitung (2),
einem ersten Mischpunkt (A) der Hauptleitung zur Aufnahme eines ersten Konzentrates (34) der mehreren Konzentrate, um mit dem Inhalt der Hauptleitung (2) vermischt zu werden und so eine Vorlösung mit einer vorbestimmten Konzentration und Substanzen des ersten Konzentrates zu erhalten,
einem zweiten Mischpunkt (C) der Hauptleitung zur Aufnahme eines zweiten Konzentrates (20) der mehreren Konzentrate, um mit dem Inhalt der Hauptleitung (2) vermischt zu werden und so eine Vorlösung mit einer vorbestimmten Konzentration von Substanzen des zweiten Konzentrates in dem Wasser zu erhalten,
gegebenenfalls weiteren Mischpunkten der Hauptleitung zur Aufnahme weiterer Konzentrate, um mit dem Inhalt der Hauptleitung (2) vermischt zu werden,
zur Bildung der einer medizinischen Einrichtung, beispielsweise einem Dialysegerät, zuzuführenden medizinischen Lösung,
**gekennzeichnet durch** einen Mischpunkt (B) zwischen dem ersten Mischpunkt (A) und dem zweiten Mischpunkt (C) zur Aufnahme einer Säure in Gasform, wobei eines der ersten und zweiten Konzentrate Bicarbonat, vorzugsweise Natriumbicarbonat, und das andere ein Calciumsalz, vorzugsweise Calciumchlorid, umfaßt.

9. Vorrichtung nach Anspruch 8, **gekennzeichnet durch** Entfernungseinrichtungen (25, 26, 27, 28) zur Entfernung von Überschußgas nach dem Vermischen und vorzugsweise auch Rezirkuliereinrichtungen zum Rezirkulieren des entfernten Gases.

10. Vorrichtung nach Anspruch 8 oder 9, **gekennzeichnet durch** eine Überwachungs- und Steuereinrichtung (30) zum Überwachen einer Eigenschaft, wie beispielsweise des pH-Wertes, der Lösung nach der Zugabe des Gases und Steuerung der Säurezufuhr in Gasform in Abhängigkeit hiervon.

11. Vorrichtung nach einem der Ansprüche 8 bis 10, **gekennzeichnet durch** einen Säuregasgenerator (13) zur Herstellung der Säure in Gasform in situ, vorzugsweise von Kohlendioxidgas, CO₂.

12. Vorrichtung nach Anspruch 11, **gekennzeichnet durch** eine Einrichtung (13) zum Mischen von Carbonat, vorzugsweise Natriumbicarbonat, mit Wasser und Säure, vorzugsweise Zitronensäure, zur Herstellung von Kohlendioxidgas, CO₂.

13. Vorrichtung nach Anspruch 11, **gekennzeichnet durch** eine Einrichtung (13) zum Erwärmen von Carbonat, vorzugsweise Natriumbicarbonat, auf eine vorbestimmte Temperatur, wie beispielsweise 50 °C, zur Herstellung von Kohlendioxidgas, CO₂.

14. Vorrichtung nach Anspruch 13, **gekennzeichnet durch** eine Abtrenneinrichtung zur Abtrennung von während der Herstellung gebildetem Wasser vor der Zufuhr zu der Hauptleitung.

15. Vorrichtung nach einem der Ansprüche 8 bis 14, **dadurch gekennzeichnet**, daß das erste Konzentrat (34) in die medizinische Lösung einzuarbeitende Metallsalze, unter anderem Calciumchlorid, umfaßt und daß das zweite Konzentrat Natriumbicarbonat umfaßt, das man durch Zuführung von Wasser durch ein trockenes Pulver von Natriumbicarbonat für kontinuierliche Herstellung der medizinischen Lösung erhalten hat.

## Revendications

1. Procédé pour la préparation d'une solution médicale, par exemple une solution de dialyse, sensiblement à partir d'eau et d'une pluralité de concentrés de substances à inclure dans ladite solution médicale, le dit procédé comprenant les étapes de :
introduction substantiellement d'eau d'une source (1) dans un conduit principal (2) ;
dosage d'un premier concentré (34) de ladite pluralité de concentrés à un premier point de mélange (A) dudit conduit principal (2) pour le mélanger au contenu dudit conduit principal (2) afin d'obtenir une présolution ayant une concentration prédéterminée de substances dudit premier concentré ;
dosage d'un deuxième concentré (20) de ladite pluralité de concentrés à un deuxième point de mélange (C) dudit conduit principal (2), pour le mélanger au contenu dudit conduit principal (2) afin d'obtenir une présolution ayant une concentration prédéterminée de substances dudit deuxième concentré dans ladite eau ;
optionnellement, dosage d' autres concentrés à d'autres points de mélange dudit conduit principal, pour les mélanger au contenu dudit conduit principal (2) ;
afin de produire ladite solution médicale à fournir à un dispositif médical, par exemple un dialyseur ;
caractérisé par :
l'introduction d'un acide sous forme de gaz à une position de mélange intermédiaire (B) entre ladite première position de mélange (A) et ladite deuxième position de mélange (C), un desdits premier et deuxième concentrés comprenant un bicarbonate, de préférence du bicarbonate de sodium, et l'autre comprenant un sel de calcium, de préférence du chlorure de calcium.

2. Procédé suivant la revendication 1, caractérisé en ce que le gaz en excès, après mélange, est éliminé à une étape de dégazage, après quoi ledit gaz éliminé est optionnellement recyclé.

3. Procédé suivant une quelconque des revendications précédentes, caractérisé en ce qu'on mesure une caractéristique de la solution après l'addition dudit gaz, par exemple le pH de la solution, et on règle l'amenée d'acide sous forme de gaz en fonction de la caractéristique mesurée.

4. Procédé suivant une quelconque des revendications précédentes, caractérisé en ce que ledit acide sous forme de gaz est préparé in situ, ledit acide sous forme de gaz étant de préférence du gaz carbonique CO₂.

5. Procédé suivant la revendication 4, caractérisé en ce qu'on prépare ledit acide sous forme de gaz, c'est-à-dire le gaz carbonique CO₂, par mélange d'un carbonate, de préférence du bicarbonate de sodium, avec de l'eau et un acide, de préférence l'acide citrique.

6. Procédé suivant la revendication 4, caractérisé en ce qu'on prépare ledit acide sous forme de gaz, c'est-à-dire le gaz carbonique CO₂, par chauffage d'un carbonate, de préférence le bicarbonate de sodium, à une température prédéterminée, par exemple 50°C.

7. Procédé suivant la revendication 6, caractérisé en ce qu'on sépare l'eau formée pendant la préparation, avant l'envoi au conduit principal.

8. Appareil pour la préparation d'une solution médicale, par exemple une solution de dialyse, sensiblement à partir d'eau et d'une pluralité de concentrés de substances à inclure dans ladite solution médicale, ledit appareil comprenant :
une entrée pour amener sensiblement de l'eau d'une source (1) à un conduit principal (2) ;
un premier point de mélange (A) dudit conduit principal pour recevoir un premier concentré (34) de la dite pluralité de concentrés à mélanger avec le contenu du dit conduit principal (2), afin d'obtenir une présolution ayant une concentration prédéterminée de substances dudit premier concentré ;
un deuxième point de mélange (C) dudit conduit principal pour recevoir un deuxième concentré (20) de ladite pluralité de concentrés à mélanger au contenu dudit conduit principal (2), afin d'obtenir une présolution ayant une concentration prédéterminée de substances dudit deuxième concentré dans ladite eau ;
optionnellement, d'autres points de mélange du dit conduit principal pour recevoir d'autres concentrés à mélanger au contenu dudit conduit principal (2) ;
afin de produire ladite solution médicale à fournir à un dispositif médical, par exemple un dialyseur ; caractérisé par :
un point de mélange intermédiaire (B) entre ledit premier point de mélange (A) et ledit deuxième point de mélange (C) pour recevoir un acide sous forme de gaz, et un desdits premier et deuxième concentrés comprend un bicarbonate, de préférence le bicarbonate de sodium, et l'autre comprend un sel de calcium, de préférence du chlorure de calcium.

9. Appareil suivant la revendication 8, caractérisé par des moyens d'élimination (25,26,27,28) pour éliminer le gaz en excès après mélange, et de préférence également par des moyens de recirculation pour recycler ledit gaz éliminé.

10. Appareil suivant la revendication 8 ou 9, caractérisé par des moyens de mesure et de réglage (30) pour mesurer une caractéristique, par exemple le pH, de la solution après l'addition dudit gaz et pour régler l'amenée d'acide sous forme de gaz en fonction de cette mesure.

11. Appareil suivant une quelconque des revendications 8 à 10, caractérisé par un générateur de gaz acide (13) pour préparer in situ ledit acide sous forme de gaz, de préférence du gaz carbonique CO₂.

12. Appareil suivant la revendication 11, caractérisé par des moyens (13) pour mélanger un carbonate, de préférence le bicarbonate de sodium, avec de l'eau et un acide, de préférence l'acide citrique, afin de préparer le gaz carbonique CO₂.

13. Appareil suivant la revendication 11, caractérisé par des moyens (13) pour le chauffage d'un carbonate, de préférence le bicarbonate de sodium, à une température prédéterminée, par exemple 50°C, afin de préparer le gaz carbonique CO₂.

14. Appareil suivant la revendication 13, caractérisé par des moyens de séparation pour séparer l'eau formée pendant la préparation, avant l'envoi au conduit principal.

15. Appareil suivant une quelconque des revendications 8 à 14, caractérisé en ce que ledit premier concentré (34) comprend des sels métalliques à inclure dans la solution médicale, notamment du chlorure de calcium, et en ce que ledit deuxième concentré comprend du bicarbonate de sodium obtenu par passage d'eau à travers une poudre sèche de bicarbonate de sodium,pour la préparation continue de la solution médicale.
